(19) Europäisches Patentamt
European Patent Office
Office européen des brevets

(11) Veröffentlichungsnummer: **0 584 461 A1**

# (12) EUROPÄISCHE PATENTANMELDUNG

(21) Anmeldenummer: **93108038.6**

(22) Anmeldetag: **17.05.93**

(51) Int. Cl.5: **C07D 233/68**, A01N 43/50

(30) Priorität: **29.05.92 DE 4217724**

(43) Veröffentlichungstag der Anmeldung: **02.03.94 Patentblatt 94/09**

(84) Benannte Vertragsstaaten: **AT BE CH DE DK ES FR GB IT LI NL PT SE**

(71) Anmelder: **BAYER AG**
**D-51368 Leverkusen(DE)**

(72) Erfinder: **Heuer, Lutz, Dr.**
**Scheuibler Strasse 83**
**W-4150 Krefeld(DE)**
Erfinder: **Kugler, Martin, Dr.**
**Am Kloster 47**
**W-5653 Leichlingen 1(DE)**
Erfinder: **Paulus, Wilfried, Dr.**
**Deswatinesstrasse 90**
**W-4150 Krefeld 1(DE)**
Erfinder: **Lorentzen, Jens-Peter, Dr., Miles Incorporated**
**Mobay Road**
**Pittsburgh, Pa 15205(US)**

## (54) 5-Halogenimidazole.

(57) Beschrieben werden 5-Halogenimidazole der Formel (I)

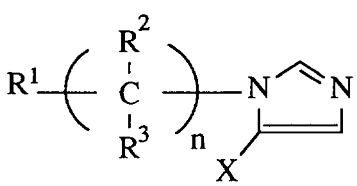 (I),

in welcher $R^1$, $R^2$, $R^3$, n und X die in der Beschreibung angegebene Bedeutung haben sowie ein Verfahren zu deren Herstellung.

Die 5-Halogenimidazole werden zur Bekämpfung von Mikroorganismen verwendet.

EP 0 584 461 A1

Die Erfindung betrifft neue 5-Halogenimidazole, ein Verfahren zu deren Herstellung sowie deren Verwendung als antimikrobielle Mittel.

5-Halogenimidazole sind zum Teil aus der SU-437, 765 bzw. Khim.-Farm. Zh, 24 56-7 (1990) und Tetrahedron Lett., 27 901 (1986) bekannt. Eine antimikrobielle Wirkung der bekannten Verbindungen wird jedoch nicht beschrieben.

Gegenstand der vorliegenden Anmeldung sind daher antimikrobielle Mittel enthaltend mindestens eine Verbindung der Formel (I)

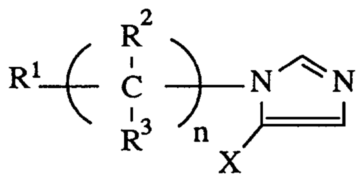 (I),

in welcher

| | | |
|---|---|---|
| X | | für Chlor oder Fluor steht und |
| $R^1$, $R^2$, $R^3$ | | unabhängig voneinander, jeweils für Wasserstoff und gegebenenfalls durch 1 bis 9 Halogenatome substituiertes $C_1$-$C_{18}$-Alkyl, $C_2$-$C_{18}$-Alkoxyalkenyl, $C_5$-$C_{11}$-Cycloalkyl, $C_2$-$C_{18}$-Alkoxyalkyl, $C_2$-$C_{18}$-Thioalkoxy, $C_2$-$C_{18}$-Alkenyl und $C_3$-$C_{18}$-Alkoxyalkylalkoxy sowie für gegebenenfalls durch Halogen, Hydroxy, und gegebenenfalls durch 1 bis 9 Halogenatome substituiertes $C_1$-$C_{10}$-Alkyl, $C_2$-$C_{10}$-Alkenyl, $C_2$-$C_{10}$-Alkoxyalkyl, $C_2$-$C_{10}$-Alkoxyalkenyl, $C_1$-$C_{10}$-Thioalkoxy, $C_1$-$C_3$-Dioxyalkyl und Aralkyloxy substituiertes Aryl und Heteroaryl steht, oder |
| $R_2$ oder $R_3$ | | zusammen mit $R^1$ einen gegebenenfalls durch Stickstoff, Schwefel oder Sauerstoff unterbrochenen Ring mit 3 bis 10 Kohlenstoffatomen bilden, und |
| n | | für die Zahl 0, 1, 2, 3 oder 4 steht. |

Alkyl, einzeln oder in zusammengesetzten Resten, steht im folgenden vorzugsweise für geradkettiges oder verzweigtes Alkyl mit 1 bis 16, insbesondere 1 bis 12 Kohlenstoffatomen wie Methyl, Ethyl, n-, i-Propyl, n-, i-, s-, t-Butyl, n-Pentyl, n-Hexyl, n-Heptyl, n-Octyl, n-Nonyl, n-Decyl, n-Undecyl, n-Dodecyl, n-Tridecyl, n-Tetradecyl, n-Pentadecyl, n-Hexadecyl sowie deren verzweigte Strukturisomere, welche gegebenenfalls durch Halogenatome wie Fluor, Chlor oder Brom substituiert sind.

Alkoxy, einzeln oder in zusammengesetzten Resten, steht im folgenden vorzugsweise für geradkettiges oder verzweigtes Alkoxy mit 1 bis 14, insbesondere 1 bis 12 Kohlenstoffatomen wie Methoxy, Ethoxy, n-, i-Propyloxy, n-, i-, s-, t-Butyloxy, n-Pentyloxy, n-Hexyloxy, n-Heptyloxy, n-Octyloxy, n-Nonyloxy, n-Decyloxy, n-Undecyloxy, n-Dedecyloxy sowie deren verzweigte Strukturisomere, welche gegebenenfalls durch Halogenatome wie Fluor, Chlor oder Brom substituiert sind.

Halogen steht im folgenden für Brom, Fluor und insbesondere Chlor.

Bevorzugt enthalten die Mittel Verbindungen der Formel (I), in welcher

| | |
|---|---|
| X | für Chlor oder Fluor steht und |
| $R^1$, $R^2$, $R^3$ | unabhängig voneinander, jeweils für Wasserstoff, gegebenenfalls durch 1 bis 3 Halogenatome substituiertes $C_5$-$C_{11}$-Alkyl, $C_5$-$C_7$-Cycloalkyl, $C_5$-$C_{11}$-Alkoxyalkyl, $C_5$-$C_{11}$-Alkenyl und $C_5$-$C_{11}$-Alkoxyalkylalkoxy, $C_5$-$C_{11}$-Alkoxyalkenyl sowie für gegebenenfalls einfach oder mehrfach durch Halogen, und gegebenenfalls durch 1 bis 3 Halogenatome substituiertes $C_1$-$C_8$-Alkyl, $C_2$-$C_8$-Alkoxy, $C_2$-$C_8$-Alkenyl, $C_1$-$C_2$-Dioxyalkyl, Phenyl und Benzyloxy substituiertes Phenyl oder Naphthyl steht, oder |
| $R_2$ oder $R_3$ | zusammen mit $R^1$ einen Ring mit 5 bis 8 Kohlenstoffatomen bilden, und |
| n | für eine Zahl 0, 1, 2, 3 oder 4 steht. |

Ganz besonders bevorzugt enthalten die Mittel Verbindungen der Formel (I), in welcher

| | |
|---|---|
| X | für Chlor steht und |
| $R^1$ | für gegebenenfalls durch 1 bis 3 Halogenatome substituiertes $C_5$-$C_{11}$-Alkyl, $C_5$-$C_{11}$-Cycloalkyl, $C_5$-$C_{11}$-Alkoxyalkyl und $C_5$-$C_{11}$-Alkoxyalkylalkoxy sowie für gegebenenfalls einfach oder zweifach durch Chlor, Fluor, Brom, $C_1$-$C_6$-Alkyl, $C_2$-$C_6$-Alkenyl, $C_1$-$C_8$-Alkoxy, Trifluormethyl, Trifluormethoxy, Difluoromethylendioxy oder Dichlorobenzyloxy substituiertes Phenyl oder Naphthyl, |
| $R^2$ und $R^3$ | für Wasserstoff stehen oder $R^2$ zusammen mit $R^1$ einen Ring mit 6, 7 oder 8 Kohlenstoff- |

atomen bilden und

n für eine Zahl 0, 1 oder 2 steht.

Gegenstand der Anmeldung sind des weiteren neue 5-Halogenimidazole der Formel (I), in welcher

X, $R^1$, $R^2$, $R^3$ und n die oben angegebenen Bedeutungen haben, ausgenommen Verbindungen, in denen n für die Zahl 0 steht und $R^1$ für Methyl, Ethyl, i-Propyl, Butyl, i-Butyl, Phenyl oder Benzyl steht.

Die neuen Verbindungen der Formel (I) werden erhalten, indem man Formylglycinamide der Formel (II)

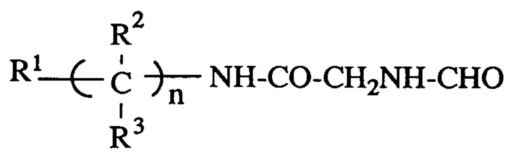 (II),

in welcher $R^1$, $R^2$, $R^3$ und n die oben angegebene Bedeutung haben, gegebenenfalls in Gegenwart eines Lösungsmittels mit einer Mischung aus Phosphorpentachlorid und Phosphorylchlorid umsetzt und anschließend in den erhaltenen Chlorimidazolen die Chloratome gegebenenenfalls gegen Fluoratome austauscht.

Die Umsetzung erfolgt vorzugsweise bei Temperaturen von 0 bis 80 °C. Der Chlor/Fluor-Austausch erfolgt vorzugsweise mittels Kaliumfluorid oder Natriumfluorid, Sulfolan oder Kaliumfluorid/Dimethylformamid nach bekannten Methoden.

Die Formylglycinamide der Formel (II)

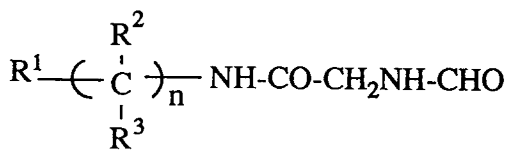 ,

in welcher $R^1$, $R^2$, $R^3$, n die oben angegebene Bedeutung haben, ausgenommen Verbindungen, in denen n für die Zahl 0 steht und $R^1$ Methyl, Ethyl, i-Propyl, Butyl, c-Hexyl, i-Butyl, Phenyl oder Benzyl bedeuten sind neu und ebenfalls Gegenstand der Anmeldung.

Die neuen Formylglycinamide der Formel (II) werden erhalten, indem man Amine der Formel (III)

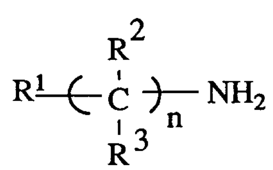 (III),

in welcher $R^1$, $R^2$, $R^3$ und n die oben angegebene Bedeutung haben,

a) mit Formylglycinester der Formel (IV)

Alkyl-O-CO-$CH_2$NH-CHO (IV)

gegebenenfalls in Gegenwart eines Lösungsmittels umsetzt, oder

b) mit Dihalogenverbindungen der Formel (V)

Hal-CO-$CH_2$-Hal (V)

gegebenenfalls in einem Lösungsmittel und gegebenenfalls Verwendung einer Hilfsbase zu Verbindun-

gen der Formel (VI)

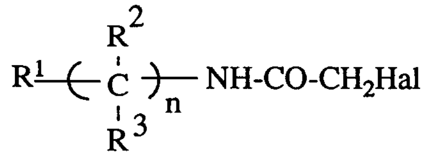

(VI)

umsetzt und anschließend diese mit Formamid in Gegenwart von Base reagieren läßt.

Die Verbindungen der Formeln (III) - (VI) sind bekannt oder können nach allgemein bekannten Methoden hergestellt werden (Organikum, Deutscher Verlag d. Wissenschaften, Berlin 1990; Tietze/Eicher, Thieme Verlag, 1981).

Als für die Reaktionen geeignete Lösungsmittel seien beispielsweise genannt: Alkohole wie Methanol, Ethanol, Propanol, Ether wie Diethylether, Dioxan, Tetrahydrofuran, Ketone wie Aceton oder Methylbutylketon; und DMF, DMSO, Acetonitril, Aromaten wie Toluol, Benzol, Chlorbenzol, Xylol. Als Basen seien genannt: $Et_3N$, $Me_3N$, Pyridin, KH, NaH, BuLi, Dimethylaminopyridin sowie Amine der Formel III.

Die erfindungsgemäßen Wirkstoffe bzw. Mittel der Formel (I) weisen eine starke Wirkung gegen Mikroorganismen auf. Sie werden im Materialschutz zum Schutz technischer Materialien verwendet: sie sind vor allem wirksam gegen Schimmelpilze, holzverfärbende und holzzerstörende Pilze und Bakterien, sowie gegen Hefen, Algen und Schleimorganismen. Beispielhaft - ohne jedoch zu limitieren - seien die folgenden Gattungen von Mikroorganismen genannt:

Alternaria wie Altenaria tenuis, Aspergillus wie Aspergillus niger und Aspergillus terteus, Aureobasidium wie Aureobasidium pullulans, Chaetomium wie Chaetomium globosum, Cladosporium wie Cladosporium herbarum, Coniophora wie Coniophora puteana, Gliocladium wie Gliocladium virens, Lentinus wie Lentinus tigrinus, Paecilomyces wie Paecilomyces variotii, Penicillium wie Penicillium brevicaule, Penicillium glaucum und Penicillium pinophilum, Polyporus wie Polyporus versicolor, Sclerophoma wie Sclerophoma pityophila, Streptoverticillium wie Streptoverticillium reticulum, Trichoderma wie Trichoderma viride, Trichophyton wie Trichophyton mentagrophytes;
Escherichia wie Escherichia coli, Pseudomonas wie Pseudomonas aereuginosa, Staphylococcus wie Staphylococcus aureus;
Candida wie Candida albicans.

Die Menge der eingesetzten Wirkstoffe ist von der Art und dem Vorkommen der Mikroorganismen der Keimzahl und von dem Medium abhängig. Die optimale Einsatzmenge kann bei der Anwendung jeweils durch Testreihen ermittelt werden. Im allgemeinen ist es jedoch ausreichend, 0,001 bis 20 Gew.-%, vorzugsweise 0,05 bis 10 Gew.-%, der Wirkstoffgemische, bezogen auf das zu schützende Material, einzusetzen.

Die neuen Wirkstoffe können als solche, in Form von Konzentraten oder allgemein üblichen Formulierungen wie Pulver, Granulate, Lösungen, Suspensionen, Emulsionen oder Pasten angewendet werden.

Die genannten Formulierungen können in an sich bekannter Weise hergestellt werden, z.B. durch Vermischen der Wirkstoffe mit Lösungs- bzw. Verdünnungsmitteln, Emulgatoren, Dispergatoren und/oder Binde- oder Fixiermitteln, gegebenenfalls Sikkative und UV-Stabilisatoren und gegebenenfalls Farbstoffen und Pigmenten sowie weiteren Verarbeitungshilfsmitteln.

Als Lösungs- bzw. Verdünnungsmittel kommen organisch-chemische Lösungsmittel oder Lösungsmittelgemische und/oder ein polares organisches Lösungsmittel oder Lösungsmittelgemische und/oder ein öliges bzw. ölartiges organisch-chemisches Lösungsmittel oder Lösungsmittelgemisch und/oder Wasser mit vorzugsweise einem Emulgator und/oder Netzmittel in Frage. Als übliche schwerflüchtige, wasserunlösliche ölige oder ölartige Lösungsmittel werden vorzugsweise die jeweiligen Mineralöle/mineralölhaltige Lösungsmittelgemische oder deren Aromatenfraktionen verwendet. Beispielhaft seien Testbenzin, Petroleum oder Alkylbenzole genannt, daneben Spindelöl und Monochlornaphthalin. Die Siedebereiche dieser schwerflüchtigen Lösemittel(gemische) überstreichen den Bereich von ca. 170 ° C bis maximal 350 ° C.

Die vorbeschriebenen schwerflüchtigen öligen oder ölartigen Lösungsmittel können teilweise oder ganz durch leichter flüchtige organisch-chemische Lösungsmittel ersetzt werden.

Zur Herstellung eines Holzschutzmittels wird vorzugsweise ein Teil des oben beschriebenen Lösungsmittels oder Lösungsmittelgemisches durch ein polares orgarisch-chemisches Lösungsmittel oder Lösungsmittelgemisches ersetzt. Vorzugsweise gelangen dabei Lösungsmittel, die Hydroxygruppen, Estergruppen, Ethergruppen oder Gemische dieser Funktionalität enthalten, zum Einsatz. Beispielhaft seien Ester oder Glykolether genannt. Als Bindemittel werden erfindungsgemäß verstanden wasserverdünnbare bzw. in

organischchemischen Lösungsmitteln löslich, dispergier- oder emulgierbare Kunstharze, bindende trocknende Öle, z.B. auf Basis von Acrylharzen, Vinylharzen, Polyesterharzen, Polyurethanharzen, Alkydharzen, Phenolharzen, Kohlenwasserstoffharzen, Silikonharzen. Das benutzte Bindemittel kann als Lösung, Emulsion oder Dispersion eingesetzt werden. Vorzugsweise werden Gemische aus Alkydharzen und trocknendem pflanzlichen Öl verwendet. Besonders bevorzugt sind Alkydharze mit einem Ölanteil zwischen 45 und 70 %.

Das erwähnte Bindemittel kann ganz oder teilweise durch ein Fixierungsmittelgemisch oder ein Weichmachergemisch ersetzt werden. Diese Zusätze sollen einer Verflüchtigung der Wirkstoffe sowie einer Kristallisation bzw. eines Ausfällens vorbeugen. Vorzugsweise ersetzen sie 0,01 bis 30 % des Bindemittels (bezogen auf 100 % des eingesetzten Bindemittels).

Die Weichmacher stammen aus den chemischen Klassen oder Phthalsäureester wie Dibutyl-, Dioctyl- oder Benzylbutylphthalat, Phosphorsäureester wie Triburylphosphat, Adipinsäureester wie Di-(2-ethylhexyl)-adipat, Stearate wie Butylstearat und Amylstearat, Oleate wie Buryloleat, Glycerinether oder höhermolekulare Glykolether, Glycerinester sowie p-Toluolsulfonsäureester.

Fixierungsmittel basieren chemisch auf Polyvinylalkylethern wie z.B. Polyvinylmethylether oder Ketonen wie Benzophenon, Ethylbenzophenon.

Als Lösungs- bzw. Verdünnungsmittels kommt vorzugsweise Wasser in Frage, gegebenenfalls in Mischung mit einem oder mehreren der obengenannten Lösungs- bzw. Verdünnungsmittel, Emulgatoren und Dispergatoren.

Technische Materialien sind erfindungsgemäß nicht lebende Materialien, die für die Verwendung in der Technik zubereitet worden sind. Beispielsweise können technische Materialien, die durch erfindungsgemäße Wirkstoffe vor mikrobieller Veränderung oder Zerstörung geschützt werden sollen, Klebstoffe, Leime, Papier und Karton, Textilien, Leder, Holz, Anstrichmittel und Kunststoffartikel, Kühlschmierstoffe und andere Materialien sein, die von Mikroorganismen befallen oder zersetzt werden können. Im Rahmen der zu schützenden Materialien seien auch Teile von Produktionsanlagen, beispielsweise Kühlwasserkreisläufe, genannt, die durch Vermehrung von Mikroorganismen beeinträchtigt werden können. Bevorzugte technische Materialien im Sinne der Erfindung sind Klebstoffe, Leime, Papiere und Kartone, Leder, Holz, Anstrichmittel, Kühlschmiermittel, wäßrige Hydraulikflüssigkeiten und Kühlkreisläufe und allgemein wäßrige funktionelle Flüssigkeiten.

Die Wirksamkeit und das Wirkungsspektrum der erfindungsgemäßen Wirkstoffe bzw. den daraus herstellbaren Mitteln, Konzentraten oder ganz allgemein Formulierungen kann erhöht werden, wenn gegebenenfalls weitere antimikrobiell wirksame Verbindungen, Fungizide, Bakterizide, Herbizide, Insektizide oder andere Wirkstoffe zur Vergrößerung des Wirkungsspektrums oder Erzielung besonderer Effekte wie z.B. dem zusätzlichen Schutz vor Insekten zugesetzt werden. Diese Mischungen können ein breiteres Wirkungsspektrum besitzen als die erfindungsgemäßen Verbindungen.

In vielen Fällen erhält man dabei synergistische Effekte, d.h. die Wirksamkeit der Mischung ist größer als die der Wirksamkeit der Einzelkomponenten. Besonders günstige Mischungspartner sind z.B. die folgenden Verbindungen:

Sulfenamide wie Dichlorfluanid (Euparen), Tolylfluanid (Methyleuparen), Folpet, Fluorfolpet;

Benzimidazole wie Carbendazim (MBC), Benomyl, Fuberidazole, Thiabendazole oder deren Salze;

Thiocyanate wie Thiocyanatomethylthiobenzothiazol (TCMTB), Methylenbisthiocyanat (MBT);

quartäre Ammoniumverbindungen wie Benzyldimethyltetradecylammoniumchlorid, Benzyl-dimethyl-dodecyl-ammoniumchlorid, Didecyl-dimethyl-ammoniumchlorid; Morpholinderivate wie $C_{11}$-$C_{14}$-4-Alkyl-2,6-dimethyl-morpholin-homologe(Tridemorph),($\pm$)-cis-4-[3-tert-Butylphenyl)-2-methylpropyl]-2,6-dimethylmorpholin (Fenpropimorph), Falimorph;

Phenole wie o-Phenylphenol, Tribromphenol, Tetrachlorphenol, Pentachlorphenol, 3-Methyl-4-chlorphenol, Dichlorophen, Chlorophen oder deren Salze;

Azole wie Triadimefon, Triadimenol, Bitertanol, Tebuconazole, Propiconazole, Azaconazole, Hexaconazole, Prochloraz, Metconazol;

Iodpropargylderivate wie Iodpropargyl-butylcarbamat (IPBC), -chlorophenylformal, -phenylcarbamat, -hexylcarbamat, -cyclohexylcarbamat, Iodpropargyloxyethylphenylcarbamat;

Iodderivate wie Diiodmethyl-p-arylsulfone z.B. Diiodmethyl-p-tolylsulfon;

Bromderivate wie Bronopol;

Isothiazolinone wie N-Methylisothiazolin-3-on, 5-Chloro-N-methylisothiazolin-3-on, 4,5-Dichloro-N-octylisothiazolin-3-on, N-Octylisothiazolin-3-on (Octhilinone);

Benzisothiazolinone, Cyclopentenisothiazoline;

Pyridine wie 1-Hydroxy-2-pyridinthion (und ihre Na-, Fe-, Ma, Zn-Salze), Tetrachlor-4-methylsulphonylpyridin;

Metallseifen wie Zinn-, Kupfer-, Zink-naphthenat, -oc-toat, -2-ethylhexanoat, -oleat, phosphat, -benzoat,

Oxide wie TBTO, $Cu_2O$, CuO, ZnO;
Organische Zinnverbindungen wie Tributylzinnnaphthenat und Tributylzinnoxid;
Dialkyldithiocarbamate wie Na- und Zn-Salze von Dialkyldithlocarbamaten, Tetramethyldiuramidisulfid (TMTD);
Nitrile wie 2,4,5,6-Tetrachlorisophthalonitril (Chlorthalonil) u.a Mikrobizide mit aktivierten Halogengruppen wie Cl-Ac, MCA, Tectamer, Bronopol, Bromidox;
Benzthiazole wie 2-Mercaptobenzothiazol; s.o. Dazomet;
Chinoline wie 8-Hydroxychinolin;
Formaldehydabspaltende Verbindungen wie Benzylalkohol-mono(poly)hemiformal, Oxazolidine, Hexahydro-s-triazine, N-Methylolchloracetamid;
Tris-N-(Cyclohexyldiazeniumdioxy)-Aluminium, N-(Cyclohexyldiazeniumdioxy)-Tributylzinn bzw. K.Salze, Bis-(N-cyclohexyl)diazinium (-dioxy- Kupfer oder Aluminium);
Als Insektizide werden bevorzugt zugesetzt:
Phosphorsäureester wie Azinphos-ethyl, Azinphos-methyl, 1-(4-Chlorphenyl)-4-(O-ethyl, S-propyl)-phosphoryloxypyrazol (TIA-230), Chlorpyrifos, Coumaphos, Demeton, Demeton-S-methyl, Diazinon, Dichlorvos, Dimethoate, Ethoprophos, Etrimfos, Fenitrothion, Fention, Heptenophos, Parathion, Parathion-methyl, Phosalone, Phoxion, Pirimiphos-ethyl, Pirimiphos-methyl, Profenofos, Prothiofos, Sulprofos, Triazophos und Trichlorphon.

Carbamate wie Aldicarb, Bendiocarb, BPMC (2-(1-Methylpropyl)phenylmethylcarbamat), Butocarboxim, Butoxycarboxim, Carbaryl, Carbofuran, Carbosulfan, Cloethocarb, Isoprocarb, Methomyl, Oxamyl, Pirimicarb, Promecarb, Propoxur und Thiodicarb.

Pyrethroide wie Allethrin, Alphamethrin, Bioresmethrin, Byfenthrin (FMC 54 800), Cycloprothrin, Cyfluthrin, Decamethrion, Cyhalothrin, Cypermethrin, Deltamethrin, Alpha-cyano-3-phenyl-2-methylbenzyl-2,2-dimethyl-3-(2-chlor-2-trifluormethylvinyl)cyclopropancarboxylat, Fenpropathrin, Fenfluthrin, Fenvalerate, Flucythrinate, Flumethrin, Fluvalinate, Permethrin und Resmethrin; Nitroimino und Nitroimide wie 1-[(6-Chlor-3-pyridinyl)-methyl]-4,5-dihydro-N-nitro-1H-imidazol-2-anin (Imidacloprid).

Organosiliciumverbindungen, vorzugsweise Dimethyl(phenyl)silylmethyl-3-phenoxy-benzylether wie z.B. Dimethyl(4-ethoxyphenyl)-silylmethyl-3-phenoxy-benzylether oder Dimethyl(phenyl)-silylmethyl-2-phenoxy-6-pyridylmethylether wie z.B. Dimethyl(9-ethoxyphenyl)-silylmethyl-2-phenoxy-6-pyridylmethylether oder (Phenyl)[3-(3-phenoxyphenyl)-propyl](dimethyl)-silane wie z.B. (4-ethoxyphenyl)-[3-(4-fluoro-3-phenoxyphenyl)propyl]dimethyl-silan.

Als andere Wirkstoffe kommen in Betracht Algizide, Molluskizide, Wirkstoffe gegen "sea animals", die sich auf z.B. Schiffsbodenanstrichen ansiedeln.

Die zum Schutz der technischen Materialien verwendeten mikrobiziden Mittel oder Konzentrate enthalten die erfindungsgemäßen Wirkstoffe in einer Konzentration von 0,001 bis 95 Gew.-%, insbesondere 0,01 bis 60 Gew.-%, daneben gegebenenfalls 0,001 bis 10 Gew.-% eines geeigneten weiteren Fungizids, Insektizids oder eines weiteren Wirkstoffs wie oben genannt.

Die erfindungsgemäßen Wirkstoffe bzw. Mittel ermöglichen in vorteilhafter Weise, die bisher verfügbaren mikrobiziden Mittel durch effektivere zu ersetzen. Sie zeigen eine gute Stabilität und haben in vorteilhafter Weise ein breites Wirkungsspektrum.

Die nachfolgenden Beispiele dienen zur Erläuterung der Erfindung ohne sie darauf zu limitieren. Teile und Prozentangaben bedeuten Gewichtsteile bzw. Gewichtsprozente.

## Herstellbeispiel

### Beispiel 1

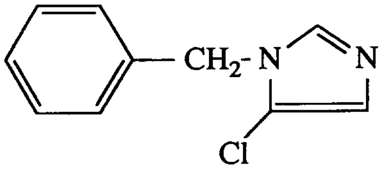

(1)

25 g Formylglycinmethylester und 22,83 g Benzylamin werden in 35 ml Methanol 3 Stunden refluxiert und anschließend eingeengt. Man erhält 41 g Formylglycinbenzamid, welches bei 10 °C in eine Mischung von 91 g Phosphorpentachlorid und 100 ml Phosphorylchlorid eingetragen werden. Nach 1 Stunde bei 25 °C

wird 150 min auf 60 ° C erwärmt, alle leicht flüchtigen Komponenten abgezogen, der Rückstand auf Eis gegeben und mit $NH_3$ auf pH 8 gebracht. Extraktion mit Chloroform ergibt nach Destillation 16,1 g (39 %) N-Benzyl-5-chlorimidazol
Die Verbindung wird durch ihr $^1H$-NMR-Spektrum (δ/ppm in $CDCl_3$ = 5,09, s) charakterisiert.

In analoger Weise und gemäß den allgemeinen Angaben in der Beschreibung werden die folgenden Verbindungen der Formel (I)

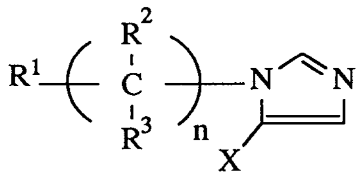

hergestellt.

Tabelle 1

| Verb. | $R^1$ | $R^2$ | $R^3$ | x | n | Physikalische Daten |
|---|---|---|---|---|---|---|
| 2 | $n-C_{11}H_{23}$ | H | H | Cl | 1 | |
| 3 | $n-C_7H_{15}$ | H | H | Cl | 1 | $\delta(CH_2-N)=$ 3,91, t |
| 4 | $n-C_9H_{19}$ | H | H | Cl | 1 | $\delta(CH_2-N)=$ 3,90, t |
| 5 | 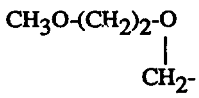 | H | H | Cl | 1 | $\delta(CH_2-N)=$ 4,13, t |
| 6 | $n-C_6H_{13}$ | H | H | Cl | 1 | $\delta(CH_2-N)=$ 4,30, t |
| 7 | $n-C_5H_{11}$ | H | H | Cl | 1 | $\delta(CH_2-N)=$ 3,90, t |
| 8 | 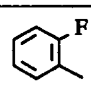 | H | H | Cl | 1 | $\delta(CH_2-N)=$ 5,15, s |
| 9 | 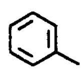 | Me | H | Cl | 1 | $\delta(CH-N)=$ 5,42, q |
| 10 | 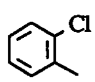 | $n-C_6H_{13}$ | H | Cl | 1 | |
| 11 | 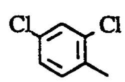 | H | H | Cl | 2 | |
| 12 | 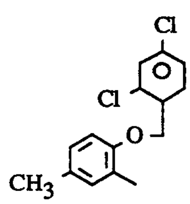 | H | H | Cl | 2 | |

Tabelle 1

| Verb. | $R^1$ | $R^2$ | $R^3$ | x | n | Physikalische Daten |
|---|---|---|---|---|---|---|
| 13 | $-CH_3$ | Me | Me | Cl | 1 | |
| 14 | 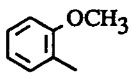 | H | H | Cl | 1 | |
| 15 | 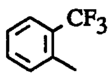 | H | H | Cl | 1 | |
| 16 | n-$C_3H_7$ | H | H | Cl | 1 | δ($CH_2$-N)= 3,91, t [Kp 0,5 = 75 - 85°C] |
| 17 | $C_2H_5$ | Me | H | Cl | 1 | |
| 18 | n-$C_4H_9$ | H | H | Cl | 1 | δ($CH_2$-N)= 3,90, t |
| 19 | 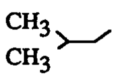 | H | H | Cl | 1 | δ ($CH_2$-N) = 3.92; dd |
| 20 | 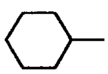 | - | - | Cl | 0 | δ(CH-N)= 3,97, m |
| 21 | 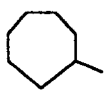 | - | - | Cl | 0 | |
| 22 | 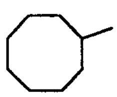 | - | - | Cl | 0 | δ(CH-N)= = 4,29, m |
| 23 | n-$C_6H_{13}$ | Me | H | Cl | 1 | |

Tabelle 1

| Verb. | $R^1$ | $R^2$ | $R^3$ | x | n | Physikalische Daten |
|---|---|---|---|---|---|---|
| 24 | $-CH_2CH(CH_3)CH_2CH_3$ | Et | H | Cl | 1 | |
| 25 | $n-C_8H_{17}$ | H | H | Cl | 1 | |
| 26 | $-(CH_2)CH(CH_3)(CH_2)_2-CH_3$ | H | Me | Cl | 1 | |
| 27 | 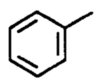 | H | H | F | 1 | |
| 28 | 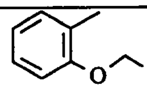 | H | H | Cl | 1 | |
| 29 | 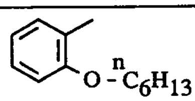 | H | H | Cl | 1 | |
| 30 | 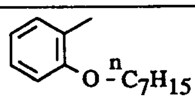 | H | H | Cl | 1 | |
| 31 | 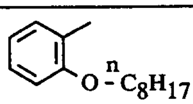 | H | H | Cl | 1 | |
| 32 | 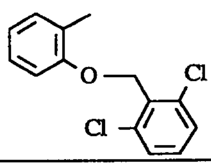 | H | H | Cl | 1 | |
| 33 | 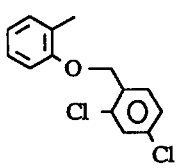 | H | H | Cl | 2 | |
| 34 | 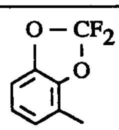 | - | - | Cl | 0 | |
| 35 | 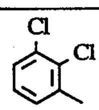 | - | - | Cl | 0 | |

Tabelle 1

| Verb. | $R^1$ | $R^2$ | $R^3$ | x | n | Physikalische Daten |
|---|---|---|---|---|---|---|
| 36 | 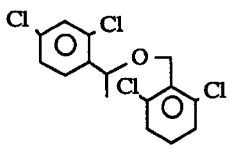 | H | H | Cl | 1 | |
| 37 | 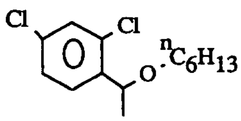 | H | H | Cl | 1 | |
| 38 | 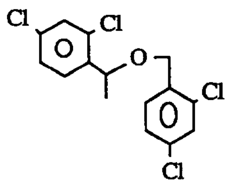 | H | H | Cl | 1 | |
| 39 | 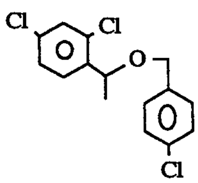 | H | H | Cl | 1 | |
| 40 | 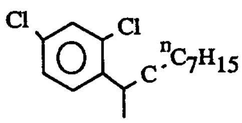 | H | H | Cl | 1 | |
| 41 | 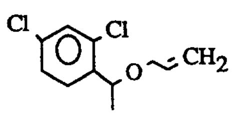 | H | H | Cl | 1 | |

Tabelle 1

| Verb. | $R^1$ | $R^2$ | $R^3$ | x | n | Physikalische Daten |
|---|---|---|---|---|---|---|
| 42 | 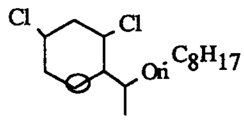 | H | H | Cl | 1 | |
| 43 | H | H | H | Cl | 1 | Kp. 82-5°C/11mm |
| 44 | 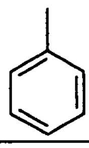 | - | - | Cl | O | |
| 45 | 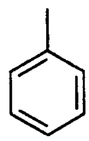 | - | - | F | O | |
| 46 | 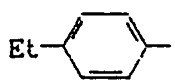 | - | - | Cl | O | δ(CH=N) 7.07s 7.64s Kp:125-137 °C/0.3 |
| 47 | 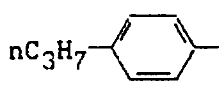 | - | - | Cl | O | |
| 48 | 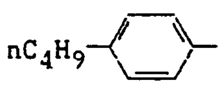 | - | - | Cl | O | |
| 49 | 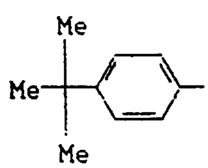 | - | - | Cl | O | |
| 50 | 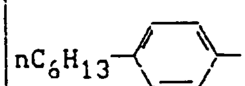 | - | - | Cl | O | |
| 51 | 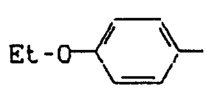 | - | - | Cl | O | |

Tabelle 1

| Comp. | $R^1$ | $R^2$ | $R^3$ | x | n | Physical data |
|---|---|---|---|---|---|---|
| 52 | 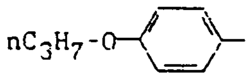 | - | - | Cl | 0 | |
| 53 | 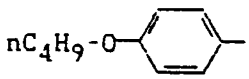 | - | - | Cl | 0 | |
| 54 | 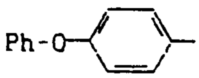 | - | - | Cl | 0 | |
| 55 | 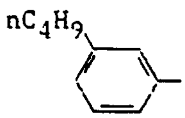 | - | - | Cl | 0 | |
| 56 | 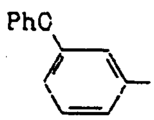 | - | - | Cl | 0 | |
| 57 | 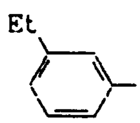 | - | - | Cl | 0 | |
| 58 | 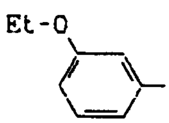 | - | - | Cl | 0 | |
| 59 | 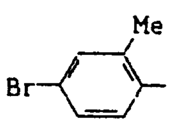 | - | - | Cl | 0 | |
| 60 | 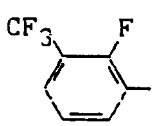 | - | - | Cl | 0 | |
| 61 | 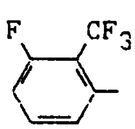 | - | - | Cl | 0 | |

Herstellung der Ausgangsverbindungen

Beispiel II-1

Zwischenprodukt II Formylglycinphenylamid

a) 40 g (0,43 mol) Anilin werden 150 ml Toluol vorgelegt und mit 33,9 g (0,3 mol) Chloracetylchlorid in 150 ml Toluol bei 10-15°C tropfenweise versetzt. Die exotherme Reaktion wird 1 h bei 25°C gehalten und dann mit 200 ml gesättigter Sodalösung 30 min lang gerührt. Es wird der gebildete Feststoff aus einer Nutsche abgetrennt und mit Ester extrahiert. Das Filrat wird mit Wasser gewaschen, getrocknet und eingeengt. Man erhält 31,9 g (63,8%) Phenylchloracetamid

b) Hiervon werden 30,0 g (0,17 mol) in 50 ml Acetonitril gelöst und tropfenweise zu einer Natriumformamidlösung gegeben, hergestellt aus 4,09 g (0,17 mol) Natrium in 75 ml absolutem Methanol und Zugabe von 75 ml absolutem Formamid sowie 50 ml Acetonitril.

Die Temperatur wird dabei 16 h bei 60°C gehalten. Nach Eingießen in Eiswasser wird mit 100 ml Methylchlorid versetzt und abgesaugt. Vertreibung des Methylenchlorids ewrgibt das gewünschte Produkt.

18,9 g (62 %) farblose Kristalle von Formylglycinphenylamid des Schmelzpunktes 124-26°C.

Anwendungsbeispiel

A. Zum Nachweis der Wirksamkeit gegen Pilze werden die minimalen Hemm-Konzentrationen (MHK) von erfindungsgemäßen Mitteln bestimmt:

Ein Agar, der unter Verwendung von Malzextrakt hergestellt wird, wird mit erfindungsgemäßen Wirkstoffen in Konzentrationen von 0,1 mg/l bis 5000 mg/l versetzt. Nach Erstarren des Agars erfolgt Kontamination mit Reinkulturen der in der Tabelle 1 aufgeführten Testorganismen. Nach 2-wöchiger Lagerung bei 28°C und 60 bis 70 % rel. Luftfeuchtigkeit wird die MHK bestimmt MHK ist die niedrigste Konzentration an Wirkstoff, bei der keinerlei Bewuchs durch die verwendete Mikrobenart erfolgt, sie ist in der nachstehenden Tabelle 1 angegeben.

Tabelle I: MHK's [mg/l] bei der Einwirkung erfindungsgemäßer Verbindungen auf Mikroorganismen

| Testorganismen | Beispiele 1 | 3 | 4 | 5 | 6 | 9 |
|---|---|---|---|---|---|---|
| Penicillium brevicaule | < 600 | < 600 | < 600 | > 800 | < 600 | < 600 |
| Chaetomium globosum | < 600 | < 600 | < 600 | > 800 | < 600 | < 600 |
| Aspergillus niger | < 600 | < 600 | > 800 | > 800 | < 600 | < 600 |
| Sclerophoma pityophila | < 600 | < 600 | < 600 | | < 600 | 800 |
| Trichoderma viride | 600 | < 600 | > 800 | | < 600 | > 800 |
| Cladosporium herberum | < 600 | < 600 | > 800 | | < 600 | < 600 |
| Alternaria tenuis | < 600 | < 600 | > 800 | | < 600 | < 600 |
| Aureobasidium pullulans | < 600 | < 600 | > 800 | | < 600 | < 600 |
| Staphylococcus aureus | < 600 | < 600 | < 600 | | < 600 | |
| Bacillus subtilis | | | | | | < 600 |

## Patentansprüche

1. Antimikrobielle Mittel enthaltend mindestens eine Verbindung der Formel (I)

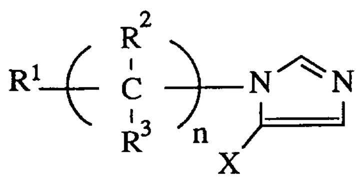 (I),

in welcher

X für Chlor oder Fluor steht und

$R^1$, $R^2$, $R^3$ unabhängig voneinander, jeweils für Wasserstoff und gegebenenfalls durch 1 bis 9 Halogenatome substituiertes $C_1$-$C_{18}$-Alkyl, $C_2$-$C_{18}$-Alkoxyalkenyl, $C_5$-$C_{11}$-Cycloalkyl, $C_2$-$C_{18}$-Alkoxyalkyl, $C_2$-$C_{18}$-Thioalkoxy, $C_2$-$C_{18}$-Alkenyl und $C_3$-$C_{18}$-Alkoxyalkylalkoxy sowie für gegebenenfalls durch Halogen, Hydroxy, und gegebenenfalls durch 1 bis 9 Halogenatome substituiertes $C_1$-$C_{10}$-Alkyl, $C_2$-$C_{10}$-Alkenyl, $C_2$-$C_{10}$-Alkoxyalkyl, $C_2$-$C_{10}$-Alkoxyalkenyl, $C_2$-$C_{10}$-Thioalkoxy, $C_1$-$C_3$-Dioxyalkyl und Aralkyloxy substituiertes Aryl und Heteroaryl steht, oder

$R_2$ oder $R_3$ zusammen mit $R^1$ einen gegebenenfalls durch Stickstoff, Schwefel oder Sauerstoff unterbrochenen Ring mit 3 bis 10 Kohlenstoffatomen bilden, und

n für die Zahl 0, 1, 2, 3 oder 4 steht.

**2.** Mittel gemäß Anspruch 1, enthaltend Verbindungen der Formel (I), in welcher

X für Chlor oder Fluor steht und

$R^1$, $R^2$, $R^3$ unabhängig voneinander, jeweils für Wasserstoff, gegebenenfalls durch 1 bis 3 Halogenatome substituiertes $C_5$-$C_{11}$-Alkyl, $C_5$-$C_7$-Cycloalkyl, $C_5$-$C_{11}$-Alkoxyalkyl, $C_5$-$C_{11}$-Alkenyl und $C_5$-$C_{11}$-Alkoxyalkylalkoxy, $C_5$-$C_{11}$-Alkoxyalkenyl sowie für gegebenenfalls einfach oder mehrfach durch Halogen, und gegebenenfalls durch 1 bis 3 Halogenatome substituiertes $C_1$-$C_8$-Alkyl, $C_2$-$C_8$-Alkoxy, $C_2$-$C_8$-Alkenyl, $C_1$-$C_2$-Dioxyalkyl, Phenyl und Benzyloxy substituiertes Phenyl oder Naphthyl steht, oder

$R_2$ oder $R_3$ zusammen mit $R^1$ einen Ring mit 5 bis 8 Kohlenstoffatomen bilden, und

n für eine Zahl 0, 1, 2, 3 oder 4 steht.

**3.** Mittel gemäß Anspruch 1, enthaltend Verbindungen der Formel (I), in welcher

X für Chlor steht und

$R^1$ für gegebenenfalls durch 1 bis 3 Halogenatome substituiertes $C_5$-$C_{11}$-Alkyl, $C_5$-$C_{11}$-Cycloalkyl, $C_5$-$C_{11}$-Alkoxyalkyl und $C_5$-$C_{11}$-Alkoxyalkylalkoxy sowie für gegebenenfalls einfach oder zweifach durch Chlor, Fluor, Brom, $C_1$-$C_6$-Alkyl, $C_2$-$C_6$-Alkenyl, $C_1$-$C_8$-Alkoxy, Trifluormethyl, Trifluormethoxy, Difluoromethylendioxy oder Dichlorobenzyloxy substituiertes Phenyl oder Naphthyl,

$R^2$ und $R^3$ für Wasserstoff stehen oder $R^2$ zusammen mit $R^1$ einen Ring mit 6, 7 oder 8 Kohlenstoffatomen bilden und

n für eine Zahl 0, 1 oder 2 steht.

**4.** 5-Halogenimidazole der Formel (I)

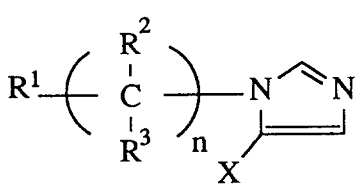 (I),

in welcher

X für Chlor oder Fluor steht und

$R^1$, $R^2$, $R^3$ unabhängig voneinander, jeweils für Wasserstoff und gegebenenfalls durch 1 bis 9 Halogenatome substituiertes $C_1$-$C_{18}$-Alkyl, $C_1$-$C_{18}$-Alkenyl, $C_1$-$C_{18}$-Cycloalkyl, $C_2$-

$C_{18}$-Alkoxyalkyl, $C_2$-$C_{18}$-Thioalkoxy, $C_2$-$C_{18}$-Alkenyl und $C_3$-$C_{18}$-Alkoxyalkylalkoxy sowie für gegebenenfalls durch Halogen, Hydroxy, und gegebenenfalls durch 1 bis 9 Halogenatome substituiertes $C_1$-$C_{10}$-Alkyl, Alkenyl, $C_2$-$C_{10}$-Alkoxyalkyl, $C_2$-$C_{10}$-Thioalkoxy, $C_1$-$C_3$-Dioxyalkyl und Aralkyloxy substituiertes Aryl und Heteroaryl steht, oder

$R_2$ oder $R_3$ zusammen mit $R^1$ einen gegebenenfalls durch Stickstoff, Schwefel oder Sauerstoff unterbrochenen Ring mit 3 bis 10 Kohlenstoffatomen bilden, und

n für die Zahl 0, 1, 2, 3 oder 4 steht.

ausgenommen Verbindungen, in denen n für die Zahl 0 steht und $R^1$ für Methyl, Ethyl, i-Propyl, Butyl, i-Butyl, Phenyl oder Benzyl steht.

**5.** Verfahren zur Herstellung von Verbindungen der Formel (I)

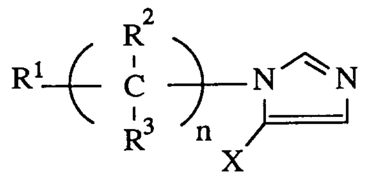 (I),

in welcher
X, $R^1$, $R^2$, $R^3$ und n die in Anspruch 4 angegebene Bedeutung haben,
dadurch gekennzeichnet, daß man Formylglycinamide der Formel (II)

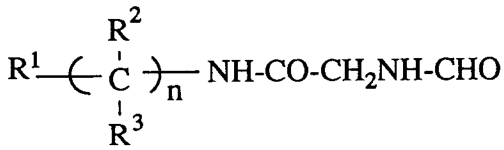 (II),

in welcher
$R^1$, $R^2$, $R^3$ und n die oben angegebene Bedeutung haben, gegebenenfalls in Gegenwart eines Lösungsmittels mit einer Mischung aus Phosphorpentachlorid und Phosphorylchlorid umsetzt und anschließend in den erhaltenen Chlorimidazolen die Chloratome gegebenenenfalls gegen Fluoratome austauscht.

**6.** Verfahren zur Bekämpfung von Mikroorganismen, dadurch gekennzeichnet, daß man 5-Halogenimidazole der Formel (I) nach Anspruch 1 auf technische Materialien einwirken läßt oder diese mit den 5-Halogenimidazolen versetzt.

**7.** Verwendung der 5-Halogenimidazole der Formel (I) nach den Ansprüchen 1 bis 5 zur Bekämpfung von Mikroorganismen zum Schutz technischer Materialien.

**8.** Formylglycinamide der Formel (II)

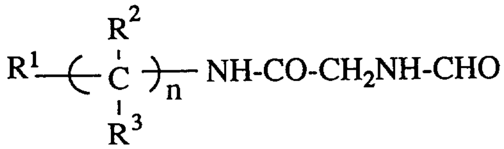 (II),

in welcher
$R^1$, $R^2$, $R^3$ und n die in Anspruch 4 angegebene Bedeutung haben.

Europäisches Patentamt

# EUROPÄISCHER RECHERCHENBERICHT

Nummer der Anmeldung

EP 93 10 8038
Seite 1

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| Y | US-A-4 237 158 (ROHM AND HAAS CO.)<br>2. Dezember 1980<br>* Spalte 1, Zeile 13 - Spalte 1, Zeile 23; Ansprüche 1-13 *<br>--- | 1-7 | C07D233/68<br>A01N43/50 |
| Y | US-A-4 115 578 (ROHM AND HAAS CO.)<br>19. September 1978<br>* Spalte 1, Zeile 10 - Spalte 1, Zeile 20; Ansprüche 1-6 *<br>--- | 1-7 | |
| Y | US-A-4 118 461 (ROHM AND HAAS CO.)<br>3. Oktober 1978<br>* Spalte 1, Zeile 10 - Spalte 1, Zeile 20; Ansprüche 1-5 *<br>--- | 1-7 | |
| Y | EP-A-0 352 675 (BASF AG)<br>31. Januar 1990<br>* Seite 27, Zeile 51 - Seite 27, Zeile 58; Ansprüche 1-8 *<br>--- | 1-7 | |
| Y | CHEMICAL ABSTRACTS, vol. 106, no. 15, 13. April 1987, Columbus, Ohio, US; abstract no. 119769e,<br>J. PERNAK ET AL. 'Effects of new quaternary iminium compounds against some bacterial and fungal strains. Part 14. Synthesis of 1-methyl-3-((n-alkylthio)methyl)-5-chloroimidazolium chlorides'<br>Seite 635 ;<br>* Zusammenfassung *<br>& Pharmazie 41 (1) 70-1 (1986)<br>---<br>-/-- | 1-7 | RECHERCHIERTE SACHGEBIETE (Int. Cl.5 )<br>C07D<br>A01N |

Der vorliegende Recherchenbericht wurde für alle Patentansprüche erstellt

| Recherchenort | Abschlußdatum der Recherche | Prüfer |
|---|---|---|
| MUENCHEN | 19 OKTOBER 1993 | HERZ C.P. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)

Europäisches Patentamt

# EUROPÄISCHER RECHERCHENBERICHT

Nummer der Anmeldung

EP 93 10 8038
Seite 2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| Y | CHEMICAL ABSTRACTS, vol. 109, no. 23, 5. Dezember 1988, Columbus, Ohio, US; abstract no. 210950e, J. PERNAK ET AL. 'Activities of new iminium compounds on selected strains of bacteria and fungi. XX. Synthesis of 1-methyl-2-alkyl-3-(n-alkoxymethyl)- and 1-methyl-3-(n-alkoxymethyl)-5-chloroimidazolium chlorides' Seite 656 ; * Zusammenfassung * & Arch. Pharm (Weinheim) 321 (4) 193-7 (1988) | 1-7 | |
| | ----- | | RECHERCHIERTE SACHGEBIETE (Int. Cl.5 ) |

Der vorliegende Recherchenbericht wurde für alle Patentansprüche erstellt

| Recherchenort | Abschlußdatum der Recherche | Prüfer |
|---|---|---|
| MUENCHEN | 19 OKTOBER 1993 | HERZ C.P. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)